# EUROPEAN PATENT APPLICATION

(11) **EP 3 949 739 A1**
(43) Date of publication of application: **09.02.2022**
(21) Application number: 20784552.0
(22) Date of filing: 25.03.2020
(51) Int. Cl.: A23C 9/123, A23C 9/13, C12N 1/20

(54) **METHOD FOR PRODUCING LACTIC ACID BACTERIUM FERMENTATION FOOD PRODUCT**

(30) Priority: 29.03.2019 JP 2019065914
(71) Applicant: Kabushiki Kaisha Yakult Honsha, Tokyo, 105-8660 (JP)
(72) Inventor: KOBAYASHI, Tatsuya, Tokyo 105-8660 (JP); SAITO, Junki, Tokyo 105-8660 (JP); SUZUKI, Takao, Tokyo 105-8660 (JP); KAMIKAWA, Tomohiro, Tokyo 105-8660 (JP)
(74) Representative: Blodig, Wolfgang
(86) International application number: PCT/JP2020/013199
(87) International publication number: WO 2020/203515

(57) **Abstract**

A proliferation promoting effect on lactic acid bacteria during production and a viability improving effect during storage are obtained by a method for producing a lactic acid bacterium fermentation food product characterized in that when a lactic acid bacterium fermentation food product is produced by inoculating and culturing lactic acid bacteria in a medium containing a milk or a milk product as a main component, a lipase degradation product of an oil or fat is added to the medium containing a milk or a milk product as a main component before culturing lactic acid bacteria, or to a fermented liquid during or after culturing.

## Description

### Technical Field

The present invention relates to a method for producing a lactic acid bacterium fermentation food product, and more particularly relates to a method for producing a lactic acid bacterium fermentation food product having a high proliferation promoting effect on lactic acid bacteria during production and also having an excellent viability improving effect on lactic acid bacteria during storage.

### Background Art

Heretofore, food and drink products containing viable cells such as yogurts and fermented milks have been widely eaten and drunk as food products having physiological functions such as an intestinal regulating function. In order to allow such food products to effectively exhibit these physiological functions, it is necessary to efficiently proliferate lactic acid bacteria during production and also maintain lactic acid bacteria without decreasing during storage, but depending on the raw materials, conditions, etc., lactic acid bacteria do not sufficiently proliferate during fermentation or die during storage resulting in a decrease in the viable cell count or the like, and there is a problem that an expected physiological function cannot be obtained.

With respect to the problem, the present applicant has already reported that the addition of oleic acid to a low-fat yogurt increases the viable cell count of lactic acid bacteria in the product and can improve the viability during storage (PTL 1). In addition, it is disclosed that by performing culturing using a medium supplemented with oleic acid or a butter fat fraction containing oleic acid, the survival rate of a microorganism in the product and in bile acid is increased (PTL 2), or by adding buttermilk, a growth promoting effect on lactic acid bacteria is obtained (PTL 3), however, there has been a demand for establishment of a technique capable of increasing the viable cell count in a product and maintaining the viable cell count in a lactic acid bacterium fermentation food product.

### Citation List

### Patent Literature

PTL 1: JP-A-2001-45968
PTL 2: JP-A-2000-102380
PTL 3: JP-A-2008-520202

### Summary of Invention

### Technical Problem

An object of the present invention is to provide a method for producing a lactic acid bacterium fermentation food product having a high proliferation promoting effect on lactic acid bacteria during production and also having an excellent viability improving effect during storage.

### Solution to Problem

As a result of intensive studies, the present inventor found that by culturing lactic acid bacteria in a medium supplemented with a lipase degradation product of an oil or fat, the proliferation of lactic acid bacteria is promoted during production, and moreover, death during storage is suppressed, and therefore, the viable cell count of lactic acid bacteria in the product can be kept high, and thus completed the present invention.

That is, the present invention is a method for producing a lactic acid bacterium fermentation food product, characterized in that when a lactic acid bacterium fermentation food product is produced by inoculating and culturing lactic acid bacteria in a medium containing a milk or a milk product as a main component, a lipase degradation product of an oil or fat is added to the medium containing a milk or a milk product as a main component before culturing lactic acid bacteria, or to a fermented liquid during or after culturing.

Further, the present invention is a method for promoting the proliferation of lactic acid bacteria, characterized in that when a lactic acid bacterium fermentation food product is produced by inoculating and culturing lactic acid bacteria in a medium containing a milk or a milk product as a main component, a lipase degradation product of an oil or fat is added to the medium containing a milk or a milk product as a main component before culturing lactic acid bacteria.

Further, the present invention is a method for improving the viability of lactic acid bacteria, characterized in that when a lactic acid bacterium fermentation food product is produced by inoculating and culturing lactic acid bacteria in a medium containing a milk or a milk product as a main component, a lipase degradation product of an oil or fat is added to the medium containing a milk or a milk product as a main component before culturing lactic acid bacteria, or to a fermented liquid during or after culturing.

Further, the present invention is a proliferation promoting agent for lactic acid bacteria or a viability improving agent for lactic acid bacteria, characterized by containing a lipase degradation product of an oil or fat as an active ingredient.

### Advantageous Effects of Invention

According to the production method of the present invention, the viable cell count in the product after production can be increased by promoting the proliferation of lactic acid bacteria during fermentation, and also the viability of lactic acid bacteria can be improved by preventing lactic acid bacteria from dying during storage. Therefore, the viable cell count of lactic acid bacteria in a lactic acid bacterium fermentation food product can be maintained within a high viable cell count range, so that the physiological functions thereof can be effectively exhibited.

### Description of Embodiments

The method for producing a lactic acid bacterium fermentation food product of the present invention is configured such that when a lactic acid bacterium fermentation food product is produced by inoculating and culturing lactic acid bacteria in a medium containing a milk or a milk product as a main component, a lipase degradation product of an oil or fat is added to the medium containing a milk or a milk product as a main component before fermenting lactic acid bacteria, or to a fermented liquid during or after culturing.

In the present invention, the lactic acid bacteria to be used in the fermentation are not particularly limited, and bacteria belonging to the genus Lactobacillus, the genus Lactococcus, the genus Streptococcus, the genus Enterococcus, and the like can be used. Specific examples of the bacteria include Lactobacillus casei, Lactobacillus acidophilus (L. acidophilus), Lactobacillus gasseri (L. gasseri), Lactobacillus helveticus (L. helveticus), Lactobacillus salivarius (L. salivarius), Lactobacillus fermentum (L. fermentum), Lactobacillus yoghurti (L. yoghurti), Lactobacillus delbrueckii subsp. bulgaricus (L. delbrueckii subsp. bulgaricus), Lactobacillus delbrueckii subsp. delbrueckii (L. delbrueckii subsp. delbrueckii), Lactobacillus gallinarum (L. gallinarum), Lactobacillus johnsoni (L. johnsoni), Lactococcus lactis subsp. Lactis, Lactococcus lactis subsp. cremoris, Lactococcus plantarum, Lactococcus raffinolactis, Streptococcus thermophilus, and Enterococcus faecium. Among these, Lactobacillus casei, Lactobacillus gasseri, and the like are preferred in terms of a proliferation promoting effect and a viability improving effect, and particularly, Lactobacillus casei is preferred. As the Lactobacillus casei, for example, Lactobacillus casei YIT 9029 strain (FERM BP-1366, date of deposit: May 18, 1987) and the like are exemplified.

Note that in the present invention, the lactic acid bacteria to be used in the fermentation also include bacteria belonging to the genus Bifidobacterium that are anaerobic bacteria in addition to those generally called lactic acid bacteria as described above. Such bacteria belonging to the genus Bifidobacterium are not particularly limited, and Bifidobacterium breve, Bifidobacterium pseudocatenulatum, Bifidobacterium bifidum, Bifidobacterium longum, Bifidobacterium infantis, Bifidobacterium adolescentis, Bifidobacterium catenulatum, Bifidobacterium angulatum, Bifidobacterium gallicum, Bifidobacterium lactis, Bifidobacterium animalis, and the like can be exemplified. Among these, Bifidobacterium bifidam, Bifidobacterium breve, and the like are preferred, and particularly, Bifidobacterium bifidam is more preferred. As the Bifidobacterium bifidum, for example, Bifidobacterium bifidum YIT 10347 (FERM BP-10613, date of deposit: June 23, 2005) and the like are exemplified.

The above-mentioned Lactobacillus casei YIT 9029 strain and Bifidobacterium bifidum YIT 10347 are deposited at present in the International Patent Organism Depositary, National Institute of Technology and Evaluation (#120, 2-5-8 Kazusakamatari, Kisarazu-shi, Chiba-ken, 292-0818, Japan).

In the present invention, one species or two or more species of the lactic acid bacteria can be used.

The above-mentioned lactic acid bacteria are cultured in a medium containing a milk or a milk product as a main component (hereinafter, sometimes simply referred to as "medium") . The milk or the milk product as the main component of the medium is not particularly limited as long as it is a milk itself or a milk product produced using a milk as a raw material, and for example, an animal milk medium composed of cow milk, goat milk, sheep milk, whole fat milk powder, defatted milk powder, fresh cream, compound cream, a whey protein concentrate (WPC), a whey protein isolate (WPI), casein, α-lactoglobulin, β-lactoglobulin, a total milk protein (TMP), or the like, or a plant-derived liquid milk such as soybean milk can be exemplified, and among these, defatted milk powder, whole fat milk powder, an animal milk such as cow milk, or a milk product produced using an animal milk as a raw material is preferably used in terms of fermentability. To the medium, in addition to the above-mentioned components, a carbohydrate such as glucose, fructose, or sucrose, another proliferation promoting agent for lactic acid bacteria such as an oolong tea extract or a tencha (sweet tea) extract, vitamins such as vitamin A, a vitamin B group, vitamin C, and vitamin E, various types of peptides, amino acids, salts of calcium, magnesium, or the like, etc. may be added.

In the present invention, to the above-mentioned medium, a lipase degradation product of an oil or fat (hereinafter sometimes simply referred to as "lipase degradation product") is added in advance before culturing lactic acid bacteria, or is added to a fermented liquid during or after culturing. Here, the lipase degradation product is preferably added to the medium before culturing or to the fermented liquid after culturing, and more preferably added to the medium before culturing. The lipase to be used is not particularly limited, but a lipase derived from a microorganism of the genus Candida, the genus Rhizopus, the genus Penicillium, or the genus Aspergillus is preferably used, and one type or two or more types among these can be used. As a commercially available product of such a lipase derived from a microorganism, for example, lipase AY "Amano" 30G (derived from Candida rugosa), Neurase F3G (derived from Rizopus niveus), lipase MER "Amano" (derived from Rizopus oryzae), lipase DF "Amano" 15 (derived from Rizopus oryzae), Lipase R "Amano" (derived from Penicillum roqueforti), Lipase A "Amano" 6 (derived from Aspergillus niger) (all manufactured by Amano Enzyme Co., Ltd.), and the like are exemplified. Among these, a lipase derived from a microorganism of the genus Candida is preferably used because it provides an excellent proliferation promoting effect during culturing of lactic acid bacteria and an excellent viability improving effect during storage.

The oil or fat to be degraded by a lipase is not particularly limited, and for example, a vegetable oil or fat, a milk fat, and the like are exemplified. One type or two or more types of these fats and oils can be used, but a vegetable oil or fat is preferred from the viewpoint of a proliferation promoting effect during culturing of lactic acid bacteria and a viability improving effect during storage, and a flavor.

The vegetable oil or fat among the oils or fats to be degraded by a lipase is not particularly limited, and for example, olive oil, sesame oil, rice bran oil, safflower oil, soybean oil, corn oil, rapeseed oil, palm oil, cottonseed oil, peanut oil, sunflower oil, or the like can be used, but among these, olive oil, sunflower oil, or the like is preferably used from the viewpoint of a proliferation promoting effect during culturing of lactic acid bacteria and a viability improving effect during storage, and a flavor. Such a vegetable oil or fat is preferably used at 100 mass% without adjusting the solid content concentration from the viewpoint of workability or the like, and is prepared by a sterilization treatment as needed.

Further, among the oils or fats to be degraded by a lipase, the milk fat is not particularly limited, and may be, for example, a material containing a milk fat such as a milk or a milk product that is the main component of the medium, and for example, cow milk, goat milk, sheep milk, whole fat milk powder, defatted milk powder, fresh cream, compound cream, or the like can be used, however, among these, fresh cream, whole fat milk powder, butter, or the like is preferably used from the viewpoint of a proliferation promoting effect during culturing of lactic acid bacteria and a viability improving effect during storage. Such a milk fat is preferably used by adjusting the fat concentration to about 3 to 50 mass% from the viewpoint of workability or the like, and is prepared by a sterilization treatment as needed.

The lipase degradation product to be used in the present invention is obtained by adding the lipase to the oil or fat to cause a reaction. The addition amount of the lipase is preferably from 0.9 to 2.4 mass%, and more preferably from 1.2 to 1.8 mass% with respect to the oil or fat. The reaction temperature is generally from 30 to 60°C, and preferably from 40 to 50°C, and the reaction is allowed to proceed generally for 4 to 48 hours, and preferably for about 12 to 24 hours at the temperature. When these are within such ranges, an excellent proliferation promoting effect during fermentation of lactic acid bacteria and an excellent viability improving effect during storage are obtained, and further, such ranges are preferred also from the viewpoint of cost and workability. After completion of the reaction, a sterilization treatment is performed as needed, however, in order to prevent solidification by the sterilization treatment in the milk fat, it is preferred to add a neutralizer to neutralize the reaction mixture before the end of the reaction. As the neutralizer, potassium hydroxide, sodium hydroxide, potassium carbonate, sodium bicarbonate, or the like is used, but when potassium carbonate or sodium bicarbonate is added, bubbles are sometimes generated, and therefore, potassium hydroxide or sodium hydroxide is preferably used. By adding such a neutralizer, the reaction mixture is neutralized to a pH of 6.5 to 7.5, preferably to a pH of 6.5 to 7.0. The neutralizer may be added at any time as long as it is after the sterilization treatment of the oil or fat to be performed prior to the addition of the lipase and before the sterilization treatment after completion of the lipase reaction. However, when the neutralizer is added at the initial stage of the lipase reaction, the reaction may not sufficiently proceed, and therefore, the neutralizer is preferably added during the period between 2 hours before completion of the reaction and before the sterilization treatment after completion of the reaction. The lipase degradation product after completion of the reaction is in the form of an aqueous solution or a paste and can be used as it is, but is preferably used after being powdered by spray drying or the like.

The lipase degradation product obtained by degrading the oil or fat by a lipase can usually contain free fatty acids and their monoglycerides, diglycerides, and triglycerides.

Such a lipase degradation product is added beforehand to the above-mentioned medium containing a milk or a milk product as a main component or added to a fermented liquid during or after culturing. The addition amount of the lipase degradation product is not particularly limited, but is preferably from 3 to 100 ppm, more preferably from 5 to 80 ppm, and particularly preferably from 5 to 40 ppm in terms of free fatty acids. In the present description, the term "free fatty acids" means free butyric acid, caproic acid, caprylic acid, capric acid, lauric acid, myristic acid, myristoleic acid, palmitic acid, stearic acid, oleic acid, and linoleic acid, and the "amount in terms of free fatty acids" is a value converted into the total content of these free fatty acids, and is obtained from the measured value of each free fatty acid content by HPLC described later. When it is within such a range, a fermentation food product having an excellent proliferation effect on lactic acid bacteria during production and an excellent viability improving effect during storage and also having an excellent flavor is obtained.

In the medium to which the lipase degradation product is added beforehand in this manner, the above-mentioned lactic acid bacteria are inoculated and cultured, or the lipase degradation product is added to a fermented liquid during or after culturing. The culturing may be performed, for example, at about 20 to 50°C for about 8 to 48 hours. Further, the culturing may be performed under an anaerobic condition as needed. Note that by performing the culturing in the medium supplemented with the lipase degradation product, the proliferation of lactic acid bacteria can be promoted, and the cell count of lactic acid bacteria in the fermented liquid after completion of the culturing can be increased to, for example, 1.2 × 10⁹ cfu/mL or more, preferably 1.5 × 10⁹ cfu/mL. The fermented liquid obtained by the culturing can be finished into a final product by subjecting it to a homogenization treatment as needed, and then adding and mixing a separately prepared syrup solution therewith, and further adding a flavor or the like thereto.

Note that in the present invention, the lactic acid bacterium fermentation food product includes drinks such as fermented milks and dairy product lactic acid bacteria drinks specified by the Ministerial Ordinance on Milk and Milk products Concerning Compositional Standards, etc., hard yogurts, soft yogurts, plain yogurts, and further kefir, cheese, and the like. In addition, the lactic acid bacterium fermentation food product of the present invention includes food and drink products using various lactic acid bacteria such as fermented milks of plain type, flavored type, fruit type, sweet type, soft type, drink type, solid (hard) type, frozen type, and the like, lactic acid bacteria drinks, kefir, cheese, and the like.

Further, in the lactic acid bacterium fermentation food product of the present invention, in addition to the syrup solution or the like, arbitrary components such as various types of other food materials, for example, various types of carbohydrates, a thickener, an emulsifier, and various types of vitamins can be blended as needed. As such food materials, specifically, carbohydrates such as sucrose, glucose, fructose, palatinose, trehalose, lactose, xylose, and malt sugar, sugar alcohols such as sorbitol, xylitol, erythritol, lactitol, Palatinit, reduced starch syrup, and reduced malt sugar starch syrup, high-intensity sweeteners such as aspartame, Somatin, sucralose, acesulfame K, and stevia, various types of thickeners (stabilizers) such as agar, gelatin, carrageenan, guar gum, xanthan gum, pectin, locust bean gum, gellan gum, carboxymethyl cellulose, soybean polysaccharides, and propylene glycol alginate, emulsifiers such as sucrose fatty acid esters, glycerin fatty acid esters, polyglycerin fatty acid esters, sorbitan fatty acid esters, and lecithin, milk fats such as cream, butter, and sour cream, acidulants such as citric acid, lactic acid, acetic acid, malic acid, tartaric acid, and gluconic acid, various types of vitamins such as vitamin A, a vitamin B group, vitamin C, and a vitamin E group, minerals such as calcium, magnesium, zinc, iron, and manganese, and flavors of yogurt type, berry type, orange type, Chinese quince type, perilla type, citrus type, apple type, mint type, grape type, apricot type, pear, custard cream, peach, melon, banana, tropical, herb type, black tea, and coffee type can be blended.

The thus obtained lactic acid bacterium fermentation food product of the present invention has excellent viability during storage, and therefore, for example, even if the fermentation food product is stored at 10°C for 3 weeks, it can maintain a viable cell rate of 150% or more, preferably 200% or more as compared to a lactic acid bacterium fermentation food product which does not use the present invention, and can exhibit a high viability rate.

As described above, the lipase degradation product of an oil or fat has an effect of promoting the proliferation of lactic acid bacteria by being added to the medium, and therefore can be utilized as a proliferation promoting agent for lactic acid bacteria by being used as an active ingredient. As the medium to which the proliferation promoting agent is added, other than the above-mentioned medium containing a milk or a milk product as an active ingredient, any medium can be used without any limitation as long as it is a known medium to be used for culturing lactic acid bacteria, and for example, MRS medium, SPC medium, and the like can be exemplified. By adding the proliferation promoting agent of the present invention in an amount of preferably 3 to 100 ppm, and more preferably 5 to 40 ppm in terms of free fatty acids to such a medium, an excellent proliferation promoting effect on lactic acid bacteria can be obtained.

Further, the lipase degradation product of an oil or fat has an effect of improving the viability of lactic acid bacteria during storage by being added to the medium or the fermented liquid during or after culturing, and therefore can be utilized as a viability improving agent for lactic acid bacteria by being used as an active ingredient. As the medium to which the viability improving agent is added, other than the above-mentioned medium containing a milk or a milk product as an active ingredient, any medium can be used without any limitation as long as it is a known medium to be used for culturing lactic acid bacteria, and for example, MRS medium, SPC medium, and the like can be exemplified. By adding the viability improving agent of the present invention in an amount of preferably 3 to 100 ppm, and more preferably 5 to 40 ppm in terms of free fatty acids to such a medium, an excellent viability improving effect on lactic acid bacteria can be obtained.

It is preferred that in the proliferation promoting agent and the viability improving agent for lactic acid bacteria, further yeast and an emulsifier are incorporated. In addition, in the proliferation promoting agent and the viability improving agent for lactic acid bacteria, a flavoring agent or any of various types of solvents may be incorporated as needed.

The yeast to be used in the proliferation promoting agent and the viability improving agent for lactic acid bacteria is not particularly limited, but for example, yeast such as high mineral content yeast is preferred. The content of the yeast in the proliferation promoting agent and the viability improving agent for lactic acid bacteria is not particularly limited, but is, for example, from 1.0 to 10 mass%, and preferably from 3.0 to 8.0 mass%.

The emulsifier to be used in the proliferation promoting agent and the viability improving agent for lactic acid bacteria is not particularly limited, but for example, a polyglycerin fatty acid ester, a sucrose fatty acid ester, a polysorbate, xanthan gum, guar gum, or the like can be used. The content of the emulsifier/thickening stabilizer in the proliferation promoting agent and the viability improving agent for lactic acid bacteria is not particularly limited, but is, for example, from 0.2 to 1.0 mass%, and preferably from 0.3 to 0.5 mass% in the case of the thickening stabilizer such as xanthan gum or guar gum.

A method for preparing the proliferation promoting agent and the viability improving agent for lactic acid bacteria is not particularly limited, but, for example, it is preferred to perform the preparation as follows: a mixture obtained by mixing a lipase degradation product of an oil or fat, a solvent such as propylene glycol, and an emulsifier, and a dispersion obtained by dispersing yeast in a solvent such as water are mixed, and the resulting mixture is further stirred and mixed.

### Examples

Hereinafter, the present invention will be described in more detail with reference to Examples, but it goes without saying that the present invention is not limited to these Examples.

The measurement of the free fatty acid content in the following Examples was performed by HPLC under the following conditions.

### (HPLC Conditions)

To 3.5 g of the sample, 1 mL of a methanol solution of tridecanoic acid (50 µg/mL) as an internal reference standard and 15 mL of acetonitrile were added, and after stirring and centrifugation, 3.5 mL of the supernatant was collected, acetonitrile was removed by a centrifugal evaporator, and the volume was made up to about 5 mL with methanol and the resulting mixture was filtered through a 0.45 µm filter. To 5 volumes of the resulting solution, 1 volume of an acetone solution (1 mg/mL) of ADAM (9-anthryldiazomethane, manufactured by Funakoshi Co., Ltd.) was added, and the resulting mixture was left to stand at room temperature in a dark place for 90 minutes or more, and analyzed by high-performance liquid chromatography. As the standard substances, a total of 9 types of butyric acid, caproic acid, caprylic acid, capric acid, lauric acid, myristic acid, palmitic acid, stearic acid, and oleic acid were used, and the sum of these 9 types of free fatty acids was taken as the total free fatty acids. The analysis was performed under the following conditions.

### (Analysis Conditions)

Column: Unison UK-C8 manufactured by Imtakt Corporation
Column temperature: 30°C
Flow rate: 1.0 mL/min
Mobile phase: Liquid A (acetonitrile/water = 65/35) and liquid B (acetonitrile/water = 85/15) were used, and elution was performed with 100% liquid A in 0-9 min, a linear gradient from 0 to 100% liquid B in 9-28 min, and 100% liquid B in 28-46 min.
Injection amount: 10 µL
Excitation wavelength: 365 nm
Fluorescence wavelength: 412 nm

### Production Example 1

### Preparation of Fresh Cream Lipase Degradation Product Paste:

3880 g of fresh cream (solid content: 51.7%) was sterilized at 98°C for 30 minutes. 15 g of lipase AY "Amano" 30G and 135 g of water were stirred and mixed, whereby a liquid A was prepared. The liquid A was added to the sterilized fresh cream, and an enzymatic reaction was allowed to proceed for 20 hours. After completion of the reaction, the reaction solution was neutralized by adding a 50 mass% potassium hydroxide aqueous solution thereto until the pH reached 6.7. After neutralization, sterilization was performed at 95°C for 15 minutes, whereby a fresh cream lipase degradation product paste was obtained. When the free fatty acid content in the fresh cream lipase degradation product paste was measured by HPLC, it was 38.38 mass%.

### Production Example 2

### Preparation of Fresh Cream Lipase Degradation Product Powder:

3880 g of fresh cream (solid content: 51.7%) was sterilized at 98°C for 30 minutes. 15 g of lipase AY "Amano" 30G and 135 g of water were stirred and mixed, whereby a liquid A was prepared. The liquid A was added to the sterilized fresh cream, and an enzymatic reaction was allowed to proceed for 20 hours. After completion of the reaction, the reaction solution was neutralized by adding a 50 mass% potassium hydroxide aqueous solution thereto until the pH reached 6.7. After neutralization, sterilization was performed at 95°C for 15 minutes, whereby a fresh cream lipase degradation product paste was obtained. 342.8 g of defatted milk powder, 306.4 g of the fresh cream lipase degradation product paste, and 1154.5 g of water were stirred and mixed, and the resulting mixture was powdered by spray drying, whereby a fresh cream lipase degradation product powder was obtained. When the free fatty acid content in the fresh cream lipase degradation product powder was measured in the same manner as in Production Example 1, it was 20.47 mass%.

### Production Example 3

### Preparation of Whole Milk Powder Lipase Degradation Product Aqueous Solution:

40 g of whole milk powder (solid content: 95%) was dissolved in 100 g of water, and the resulting solution was sterilized at 98°C for 30 minutes. 0.12 g of lipase AY "Amano" 30G was added thereto, and an enzymatic reaction was allowed to proceed at 50°C for 20 hours. After completion of the reaction, the reaction solution was neutralized by adding a 50 mass% potassium hydroxide aqueous solution thereto until the pH reached 6.7. After neutralization, sterilization was performed at 95°C for 15 minutes, whereby a whole fat milk powder lipase degradation product aqueous solution was obtained. When the free fatty acid content in the whole fat milk powder lipase degradation product aqueous solution was measured in the same manner as in Production Example 1, it was 3.59 mass%.

### Production Example 4

### Preparation of Whole Fat Milk Powder Lipase Degradation Product Powder:

40 g of whole fat milk powder (solid content: 95%) was dissolved in 157.56 g of water, and the resulting solution was sterilized at 98°C for 30 minutes, whereby a liquid A was prepared. 0.12 g of lipase AY "Amano" 30G and 0.108 g of water were stirred and mixed, whereby a liquid B was prepared. The liquid B was added to the liquid A, and an enzymatic reaction was allowed to proceed at 50°C for 20 hours. After completion of the reaction, the reaction solution was neutralized by adding a 50 mass% potassium hydroxide aqueous solution thereto until the pH reached 6.7. After neutralization, sterilization was performed at 95°C for 15 minutes. After cooling, the resulting mixture was powdered by spray drying, whereby a whole fat milk powder lipase degradation product powder was obtained. When the free fatty acid content in the whole fat milk powder lipase degradation product powder was measured in the same manner as in Production Example 1, it was 15.55 mass%.

### Production Example 5

### Preparation of Butter Lipase Degradation Product Paste:

3000 g of butter (solid content: 83%) was melted at 50°C, and thereafter sterilized at 98°C for 30 minutes . 37.5 g of lipase AY "Amano" 30G and 337.5 g of water were stirred and mixed, whereby a liquid A was prepared. The liquid A was added to the sterilized butter, and an enzymatic reaction was allowed to proceed at 50°C for 20 hours. After completion of the reaction, the reaction solution was sterilized at 95°C for 15 minutes, whereby a butter lipase degradation product paste was obtained. When the free fatty acid content in the butter lipase degradation product paste was measured by HPLC, it was 47.1 mass%.

### Production Example 6

### Preparation of Butter Lipase Degradation Product Powder:

3000 g of butter (solid content: 83%) was melted at 50°C, and thereafter sterilized at 98°C for 30 minutes . 37.5 g of lipase AY "Amano" 30G and 337.5 g of water were stirred and mixed, whereby a liquid A was prepared. The liquid A was added to the sterilized butter, and an enzymatic reaction was allowed to proceed at 50°C for 20 hours. After completion of the reaction, the reaction solution was sterilized at 95°C for 15 minutes. After cooling, the reaction solution was powdered by spray drying, whereby a butter lipase degradation product paste was obtained. 25 g of defatted milk powder, 0.2 g of potassium hydroxide, 10 g of the butter lipase degradation product paste, and 114.8 g of water were stirred and mixed, and the resulting mixture was powdered by spray drying, whereby a butter lipase degradation product powder was obtained. When the free fatty acid content in the butter lipase degradation product powder was measured in the same manner as in Production Example 1, it was 14.6 mass%.

### Example 1

### Production of Lactic Acid Bacterium Fermentation Food Product:

To a medium containing 16 w/v% of defatted milk powder, 10 w/v% of glucose, and 0.2 w/v% of an oolong tea extract, the fresh cream lipase degradation product paste obtained in Production Example 1 was added so that the total free fatty acid content was 16.5 ppm, followed by sterilization at 100°C for 62 minutes, whereby a culture medium was obtained. Separately, Lactobacillus casei YIT 9029 strain was inoculated at 0.5 v/v% in a 10 w/v% defatted milk powder solution and cultured at 37°C for 24 hours, whereby a culture solution was obtained. The culture solution was inoculated at 0.5 v/v% in the culture medium and cultured at 35°C, and the culturing was completed when the acidity reached 24 mL/9 g, whereby a lactic acid bacterium fermentation food product was obtained. Further, for comparison, a lactic acid bacterium fermentation food product was obtained in the same manner as described above except that a medium to which no lipase degradation product was added was used. The viable cell count (cfu/mL) in each of the lactic acid bacterium fermentation food products at completion of the culturing and after storage at 10°C for 14 days and 21 days was measured in BCP medium. These results are shown in Table 1.

**[Table 1]**

| Viable cell count | No addition | Fresh cream lipase degradation product paste |
|---|---|---|
| at completion of culturing | 7.9 × 10⁸ | 1.2 × 10⁹ |
| after storage for 14 days | 7.3 × 10⁸ | 1.1 × 10⁹ |
| after storage for 21 days | 6.0 × 10⁸ | 9.8 × 10⁸ |

When culturing lactic acid bacteria in the medium, by adding the fresh cream lipase degradation product paste to the medium, the viable cell count at completion of the culturing and the viable cell count after storage significantly increased as compared to the case where no lipase degradation product was added to the medium.

### Example 2

### Production of Lactic Acid Bacterium Fermentation Food Product:

To a medium containing 16 w/v% of defatted milk powder, 2.2 w/v% of glucose, 5.1 w/v% of fructose, and 0.2 w/v% of an oolong tea extract, each of the fresh cream lipase degradation product paste obtained in Production Example 1, the fresh cream lipase degradation product powder obtained in Production Example 2, the whole milk powder lipase degradation product aqueous solution obtained in Production Example 3, and the whole milk powder lipase degradation product powder obtained in Production Example 4 was added so that the total free fatty acid content was 27.7 ppm, followed by sterilization at 100°C for 62 minutes, whereby a culture medium was obtained. Separately, Lactobacillus casei YIT 9029 strain was inoculated at 0.5 v/v% in a 10 w/v% defatted milk powder solution and cultured at 37°C for 24 hours, whereby a culture solution was obtained. The culture solution was inoculated at 0.5 v/v% in the culture medium and cultured at 35°C, and the culturing was completed when the acidity reached 24 mL/9 g, whereby a lactic acid bacterium fermentation food product was obtained. The viable cell count (cfu/mL) in each of the lactic acid bacterium fermentation food products at completion of the culturing and after storage at 10°C for 14 days was measured in BCP medium. Further, the flavor of each of the lactic acid bacterium fermentation food products was evaluated according to the following evaluation criteria. These results are shown in Table 2.

**[Table 2]**

| Type of lipase degradation product | Fresh cream lipase degradation product paste | Fresh cream lipase degradation product powder | Whole milk powder lipase degradation product aqueous solution | Whole milk powder lipase degradation product powder |
|---|---|---|---|---|
| Viable cell count at completion of culturing | 1.3 × 10⁹ | 1.3 × 10⁹ | 1.3 × 10⁹ | 1.2 × 10⁹ |
| Viable cell count after storage for 14 days | 1.2 × 10⁹ | 1.2 × 10⁹ | 1.3 × 10⁹ | 1.3 × 10⁹ |
| Flavor | B | B | B | B |

### <Evaluation Criteria for Flavor>

### (Evaluation): (Contents)

- A:: very good
- B:: good
- C:: slightly bad
- D:: bad

Even when adding any of the lipase degradation products, the proliferation of lactic acid bacteria in the production was promoted to the same extent as in Example 1, and also the viability during storage was improved. Further, also with respect to the flavor, food products having favorable flavor were obtained.

### Example 3

### Production of Lactic Acid Bacterium Fermentation Food Product:

To a medium containing 16 w/v% of defatted milk powder, 2.2 w/v% of glucose, 5.1 w/v% of fructose, and 0.2 w/v% of an oolong tea extract, each of the fresh cream lipase degradation product paste obtained in Production Example 1, the butter lipase degradation product paste obtained in Production Example 5, and the butter lipase degradation product powder obtained in Production Example 6 was added so that the total free fatty acid content was 53.5 ppm, followed by sterilization at 100°C for 62 minutes, whereby a culture medium was obtained. Separately, Lactobacillus casei YIT 9029 strain was inoculated at 0.5 v/v% in a 10 w/v% defatted milk powder solution and cultured at 37°C for 24 hours, whereby a culture solution was obtained. The culture solution was inoculated at 0.5 v/v% in the culture medium and cultured at 35°C, and the culturing was completed when the acidity reached 24 mL/9 g, whereby a lactic acid bacterium fermentation food product was obtained. Further, for comparison, a lactic acid bacterium fermentation food product was obtained in the same manner as described above except that a medium to which no lipase degradation product was added was used. The viable cell count (cfu/mL) in each of the lactic acid bacterium fermentation food products at completion of the culturing and after storage at 10°C for 14 days and 21 days was measured in BCP medium. Further, the percentage of the viable cell count in the case of using each of the lipase degradation products was calculated by assuming the viable cell count in the lactic acid bacterium fermentation food product to which no lipase degradation product was added as 100%. In addition, the flavor of each of the lactic acid bacterium fermentation food products was evaluated according to the same evaluation criteria as in Example 2. These results are shown in Table 3.

**[Table 3]**

| Type of lipase degradation product | None | Fresh cream lipase degradation product paste | Butter lipase degradation product paste | Butter lipase degradation product powder |
|---|---|---|---|---|
| Addition amount of lipase degradation product* | 0 | 53.5 ppm | 53.5 ppm | 53.5 ppm |
| Viable cell count at completion of culturing | 1.1 × 10⁹ (100%) | 1.7 × 10⁹ (155%) | 1.7 × 10⁹ (155%) | 1.6 × 10⁹ (145%) |
| Viable cell count after storage for 14 days | 9.6 × 10⁸ (100%) | 1.6 × 10⁹ (167%) | 1.6 × 10⁹ (167%) | 1.6 × 10⁹ (167%) |
| Viable cell count after storage for 21 days | 7.4 × 10⁸ (100%) | 1.5 × 10⁹ (203%) | 1.6 × 10⁹ (216%) | 1.6 × 10⁹ (216%) |
| Flavor | B | C | C to B | A |

| | | | | |
|---|---|---|---|---|
| *: Total free fatty acid content | | | | |

When culturing lactic acid bacteria in the medium, by adding the lipase degradation product of a milk fat to the medium, the viable cell count at completion of the culturing and the viable cell count after storage significantly increased as compared to the case where no lipase degradation product was added to the medium.

### Production Example 7

### Preparation of Vegetable Oil or Fat Lipase Degradation Product Paste:

The raw materials were mixed in a 250 mL heat-resistant bottle according to the formulation shown in Table 4, and thereafter stirred at 50°C for 20 hours. After stirring, the enzyme was inactivated by heating at 90 to 95°C for 15 minutes. The resulting material was solidified in a refrigerator and then melted at 50°C, and a supernatant oil layer was collected and formed into a vegetable oil or fat lipase degradation product paste. The amount of free fatty acids in each of the vegetable oil or fat lipase degradation product pastes was measured by HPLC. The results are also shown in Table 5.

**[Table 4]**

| | |
|---|---|
| Vegetable oil or fat | 175.0 g |
| Lipase AY "Amano" 30G | 2.5 g |
| Water | 22.5 g |
| Total | 200.0 g |

**[Table 5]**

| Vegetable oil or fat | Amount of free fatty acids (g/kg) |
|---|---|
| Olive oil | 691 |
| Sunflower oil | 700 |

### Production Example 8

### Preparation of Vegetable Oil or Fat Lipase Degradation Product Powder:

Among the vegetable oil or fat lipase degradation product pastes obtained in Production Example 7, the vegetable oil or fat lipase degradation product paste obtained using sunflower oil was powdered by spray drying, whereby a sunflower oil lipase degradation product powder was obtained.

### Example 4

### Production of Lactic Acid Bacterium Fermentation Food Product:

To a medium containing 16 w/v% of defatted milk powder, 2.2 w/v% of glucose, 5.1 w/v% of fructose, and 0.2 w/v% of an oolong tea extract, each of the olive oil lipase degradation product paste and the sunflower oil lipase degradation product paste obtained in Production Example 7, and the sunflower oil lipase degradation product powder obtained in Production Example 8 was added so that the total free fatty acid content was 17.4 ppm, followed by sterilization at 100°C for 62 minutes, whereby a culture medium was obtained. Separately, Lactobacillus casei YIT 9029 strain was inoculated at 0.5 v/v% in a 10 w/v% defatted milk powder solution and cultured at 37°C for 24 hours, whereby a culture solution was obtained. The culture solution was inoculated at 0.5 v/v% in the culture medium and cultured at 35°C, and the culturing was completed when the acidity reached 24 mL/9 g, whereby a lactic acid bacterium fermentation food product was obtained. Further, for comparison, a lactic acid bacterium fermentation food product was obtained in the same manner as described above except that a medium to which no lipase degradation product was added was used. The viable cell count (cfu/mL) in each of the lactic acid bacterium fermentation food products at completion of the culturing and after storage at 10°C for 14 days and 21 days was measured in BCP medium. Further, the percentage of the viable cell count in the case of using each of the lipase degradation products was calculated by assuming the viable cell count in the lactic acid bacterium fermentation food product to which no lipase degradation product was added as 100%. In addition, the flavor of each of the lactic acid bacterium fermentation food products was evaluated according to the same evaluation criteria as in Example 2. These results are shown in Table 6.

**[Table 6]**

| Type of lipase degradation product | None | Olive oil lipase degradation product paste | Sunflower oil lipase degradation product paste | Sunflower oil lipase degradation product powder |
|---|---|---|---|---|
| Addition amount of lipase degradation product* | 0 | 17.4 ppm | 17.4 ppm | 17.4 ppm |
| Viable cell count at completion of culturing | 1.1 × 10⁹ (100%) | 1.9 × 10⁹ (173%) | 1.7 × 10⁹ (155%) | 1.7 × 10⁹ (155%) |
| Viable cell count after storage for 14 days | 9.6 × 10⁸ (100%) | 1.5 × 10⁹ (156%) | 1.5 × 10⁹ (156%) | 1.7 × 10⁹ (177%) |
| Viable cell count after storage for 21 days | 7.4 × 10⁸ (100%) | 1.5 × 10⁹ (203%) | 1.5 × 10⁹ (203%) | 1.5 × 10⁹ (203%) |
| Flavor | B | A | A | A |

| | | | | |
|---|---|---|---|---|
| *: Total free fatty acid content | | | | |

When culturing lactic acid bacteria in the medium, by adding the lipase degradation product of a vegetable oil or fat to the medium, the viable cell count at completion of the culturing and the viable cell count after storage significantly increased as compared to the case where no lipase degradation product was added to the medium. Further, a significant effect could be confirmed at a low concentration as compared to the milk fat-derived oil or fat lipase degradation product. In addition, it was found that the lipase degradation product of a vegetable oil or fat improves also the flavor of the lactic acid bacterium fermentation food product.

### Production Example 9

### Preparation of Proliferation Promoting Agent/Viability Improving Agent for Lactic Acid Bacteria:

16.2 g of the olive oil lipase degradation product prepared in Production Example 7, 87.5 g of propylene glycol, and 4.0 g of xanthan gum were stirred and mixed, whereby a liquid A was prepared. Separately, 649.7 g of water, 41.8 g of yeast extract, 200.0 g of a flavoring agent, and 0.8 g of citric acid were stirred and mixed, whereby a liquid B was prepared. The liquid A and the liquid B were stirred and mixed, whereby a proliferation promoting agent/viability improving agent for lactic acid bacteria was obtained.

The proliferation promoting agent/viability improving agent for lactic acid bacteria could be stably stored at 10°C or lower for 1 month.

### Example 5

### Production of Lactic Acid Bacterium Fermentation Food Product:

In a medium obtained by heat sterilization of an aqueous solution containing 14 w/w% of whole milk powder, 4 w/w% of defatted milk powder, and 0.1 w/w% of milk peptide (LE80GF-US, manufactured by Nippon Shinyaku Co., Ltd.) at 135°C for 3 seconds, a starter of Bifidobacterium bifidum YIT 10347 strain was inoculated so that the initial cell count was about 2 × 10⁷ cfu/mL and cultured at 37°C in the air atmosphere until the pH reached 4.8 to 4.9, whereby a bifidobacteria fermented liquid was obtained. Separately, to an aqueous solution containing 7 w/w% of sucrose and 1 w/w% of sodium carboxymethyl cellulose (CMC122Y, manufactured by Daicel Corporation), the whole milk powder lipase degradation product powder obtained in Production Example 4 was added so that the total free fatty acid content was 45 ppm, followed by heat sterilization at 121°C for 3 seconds, whereby a syrup was obtained. 40 parts by mass of the thus obtained bifidobacteria fermented liquid was subjected to a homogenization treatment at 15 MPa, and thereafter added and mixed in 60 parts by mass of the syrup, whereby a lactic acid bacterium fermentation food product containing bifidobacteria was obtained. The viable cell count (cfu/mL) of the Bifidobacterium bifidum YIT 10347 strain in the lactic acid bacterium fermentation food product containing bifidobacteria at completion of the culturing and after storage at 10°C for 21 days was measured in TOS medium. Further, when the percentage of the viable cell count in the case of using each of the lipase degradation products was calculated by assuming the viable cell count in the lactic acid bacterium fermentation food product to which no lipase degradation product was added as 100%, the viable cell count after storage for 21 days was 233%. In addition, when the flavor of each of the fermentation food products was evaluated according to the same evaluation criteria as in Example 2, the flavor was evaluated as B. These results are shown in Table 7.

**[Table 7]**

| Type of lipase degradation product | None | Whole milk powder lipase degradation product powder |
|---|---|---|
| Addition amount of lipase degradation product* | 0 | 45 ppm |
| Viable cell count at completion of culturing | 4.0 × 10⁸ (100%) | 4.0 × 10⁸ (100%) |
| Viable cell count after storage for 21 days | 2.1 × 10⁷ (100%) | 4.9 × 107 (233%) |
| Flavor | B | B |

| | | |
|---|---|---|
| *: Total free fatty acid content | | |

When producing the lactic acid bacterium fermentation food product containing bifidobacteria, by adding the lipase degradation product to the fermented liquid after culturing, the viable cell count after storage significantly increased as compared to the case where no lipase degradation product was added to the fermented liquid.

### Industrial Applicability

According to the production method of the present invention, a proliferation promoting effect on lactic acid bacteria during fermentation and a viability improving effect during storage are obtained, and therefore, the viable cell count in the lactic acid bacterium fermentation food product can be maintained within a high viable cell count range, so that the physiological functions thereof can be effectively exhibited. Accordingly, the method of the present invention is useful as a method for producing a functional food product or the like.

## Claims

1. A method for producing a lactic acid bacterium fermentation food product, **characterized in that** when a lactic acid bacterium fermentation food product is produced by inoculating and culturing lactic acid bacteria in a medium containing a milk or a milk product as a main component, a lipase degradation product of an oil or fat is added to the medium containing a milk or a milk product as a main component before culturing lactic acid bacteria, or to a fermented liquid during or after culturing.

2. The method for producing a lactic acid bacterium fermentation food product according to claim 1, wherein the oil or fat is a vegetable oil or fat.

3. The method for producing a lactic acid bacterium fermentation food product according to claim 2, wherein the vegetable oil or fat is olive oil or sunflower oil.

4. The method for producing a lactic acid bacterium fermentation food product according to claim 1, wherein the oil or fat is a milk fat.

5. The method for producing a lactic acid bacterium fermentation food product according to claim 4, wherein the milk fat is derived from butter, fresh cream, or whole milk powder.

6. The method for producing a lactic acid bacterium fermentation food product according to any one of claims 1 to 5, wherein the medium containing a milk or a milk product as a main component is a medium containing, as a main component, an animal milk or a milk product produced using an animal milk as a raw material.

7. The method for producing a lactic acid bacterium fermentation food product according to any one of claims 1 to 6, wherein the lactic acid bacteria are Lactobacillus casei.

8. A method for promoting the proliferation of lactic acid bacteria, **characterized in that** when a lactic acid bacterium fermentation food product is produced by inoculating and culturing lactic acid bacteria in a medium containing a milk or a milk product as a main component, a lipase degradation product of an oil or fat is added to the medium containing a milk or a milk product as a main component before culturing lactic acid bacteria.

9. A method for improving the viability of lactic acid bacteria, **characterized in that** when a lactic acid bacterium fermentation food product is produced by inoculating and culturing lactic acid bacteria in a medium containing a milk or a milk product as a main component, a lipase degradation product of an oil or fat is added to the medium containing a milk or a milk product as a main component before culturing lactic acid bacteria, or to a fermented liquid during or after culturing.

10. A proliferation promoting agent for lactic acid bacteria, **characterized by** comprising a lipase degradation product of an oil or fat as an active ingredient.

11. The proliferation promoting agent for lactic acid bacteria according to claim 10, further comprising yeast and an emulsifier.

12. A viability improving agent for lactic acid bacteria, **characterized by** comprising a lipase degradation product of an oil or fat as an active ingredient.

13. The viability improving agent for lactic acid bacteria according to claim 12, further comprising yeast and an emulsifier.
